(19) Europäisches Patentamt
European Patent Office
Office européen des brevets

(11) Publication number: **0 171 227**
**B1**

# (12) EUROPEAN PATENT SPECIFICATION

(45) Date of publication of patent specification: **02.11.88**

(51) Int. Cl.$^4$: **A 61 K 31/135**

(21) Application number: **85305253.8**

(22) Date of filing: **24.07.85**

## (54) Use of propiophenone compound.

(30) Priority: **25.07.84 US 634451**
**22.03.85 US 715078**

(43) Date of publication of application:
**12.02.86 Bulletin 86/07**

(45) Publication of the grant of the patent:
**02.11.88 Bulletin 88/44**

(84) Designated Contracting States:
**AT BE CH DE FR GB IT LI LU NL SE**

(56) References cited:
**US-A-4 347 257**
**US-A-4 425 363**
**US-A-4 435 449**

**CHEMICAL ABSTRACTS, vol. 91, no. 9, 27th August 1979, page 444, no. 72808w, Columbus, Ohio, US; C. VILLANI et al.: "Plasma prolactin (PRL), FSH, LH and testosterone (T) in 71 male patients with sexual disturbances", PROC. SERONO SYMP. 1978, 22, 457-64**

(73) Proprietor: **THE WELLCOME FOUNDATION LIMITED**
**183-193 Euston Road**
**London NW1 2BP (GB)**

(72) Inventor: **Stern, Warren Charles**
**8904 Willow Wood Court**
**Raleigh North Carolina 27612 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 171 227 B1

**Description**

This invention is directed to a method of treatment of psychosexual dysfunction in human beings by the administration of the compound of the formula (I)

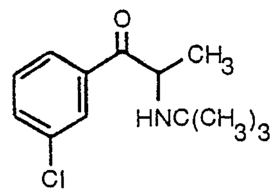

(I)

or a pharmaceutically acceptable salt thereof in a non-toxic, therapeutic amount to a human being in need thereof.

Psychosexual dysfunctions which are treated according to this invention are those in which a physical disorder or another Axis I mental disorder, e.g., major depression, is not the primary cause of disturbance in sexual function. In these situations, a psychosexual dysfunction is not properly diagnosed and this invention is not directed to those conditions in which the primary diagnosis is not psychosexual dysfunction.

The particular psychosexual dysfunctions treatable by the methods disclosed herein are:

1. Inhibited (absent/reduced) Sexual Desire (commonly referred to as inhibited libido)
2. Inhibited (absent/reduced) Sexual Excitement
3. Inhibited (absent/reduced) Female Orgasm
4. Inhibited (absent/reduced) Male Orgasm
5. Premature Ejaculation
6. Functional Dyspareunia
7. Functional Vaginismus
8. Atypical Psychosexual Dysfunction

The diagnosis for each of the conditions and a general background of psychosexual dysfunction is set forth in the text *Diagnostic and Statistical Manual of Mental Disorders* (Third Edition), American Psychiatric Association, Washington, D.C., APA, 1980, Library of Congress Catalogue Number 79-055868, Copyright American Psychiatric Association, 1980, pages 275 to 283 and in particular paragraphs 302.71, 302.72, 302.73, 302.74, 302.75, 302.76, 306.51 and 302.70 set forth on these pages 278 to 283.

There is no accepted pharmacological treatment of psychosexual dysfunction, currently accepted treatment consisting of various forms of psychotherapy and behavior therapy. The effectiveness of such treatment is quite variable, and since it tends to be both prolonged and expensive it is not accessible to many sufferers. The incidence of psychosexual dysfunction in the United States of America is not known precisely, but it is estimated to be extremely common. In males the incidence increases with age to a level as high as 75% in those over 65 years old.

The compound of formula (I) as its hydrochloride salt (bupropion hydrochloride) has been shown to be effective in treating human psychosexual dysfunction in a placebo-controlled clinical trial involving non-depressed patients and to exhibit few side-effects; however, the biological activity resides in the base and the identity of the acid is of less importance.

In U.S. Patents Nos. 3,819,706 and 3,885,046, the compound of formula (I) (chemically named m-chloro-α-t-butylaminopropiophenone) and salts thereof are disclosed as being antidepressants.

The compound of formula (I) (the active ingredient) or a pharmaceutically acceptable salt thereof is preferably administered in unit dosage form to the human being under treatment.

A pharmaceutical composition containing the compound of formula (I), or a pharmaceutically acceptable salt thereof, may be presented in discrete units such as cachets, tablets, capsules, ampules or suppositories, each containing the compound or salt in an effective amount for the treatment of psychosexual dysfunction or an appropriate fraction of such amount.

As an example, for the treatment of human beings having one or more particular types of psychosexual dysfunction, the preferred unit dosage of the compound of formula (I) or salt thereof (calculated as the base) for oral administration or for rectal administration, for example as a suppository, is about 15 milligrams to 500 milligrams, preferably 15 milligrams to 350 milligrams, the most preferred unit dosage being in the range 25 milligrams to 300 milligrams taken two or three times a day. Therapeutic (effective) dosage in humans is preferably 1 to 15 mg/kg (orally) per day. The most preferred unit dose is 135 mg in terms of base taken 2 or 3 times per day. Treatment is given on a continuous basis to a person already diagnosed as having psychosexual dysfunction. All the above doses are expressed in terms of the weight of the compound of formula (I) in the form of its base, but as will be appreciated from the foregoing information, it may be administered in the form of a pharmaceutically acceptable salt thereof. Parenteral administration may be used and in this case the dose would be about one half the above-indicated oral dosage.

The compound of formula (I) or a pharmaceutically acceptable salt thereof may be presented as an oral unit preparation (for example as a cachet, tablet or capsule) containing in addition one or more pharmaceutically acceptable carriers which may take the form of solid diluents such as lactose, cornstarch, micronized silica gel as well as other excipients known in the art.

It should be understood that in addition to the aforementioned ingredients, the pharmaceutical compositions of this invention may include one or more additional ingredients, e.g., pharmaceutically acceptable carriers such as diluents, buffers, flavouring agents, binders, surface active agents, thickeners, lubricants, preservatives and the like. The formulations may be prepared by admixture of the ingredients and, if necessary, shaping of the resulting mass, and filling into suitable containers.

The compound of formula (I) is preferably presented for use as a pharmaceutically acceptable salt. Examples of some of the pharmaceutically acceptable salts which can be utilized are salts of the following acids: hydrochloric, sulfuric, phosphoric and toluenesulphonic acids.

Reference should be had to U.S. Patents 3,819,706 and 3,885,046, which are incorporated herein by reference thereto for a description of the preparation of the compound of formula (I), salts thereof and tablets, capsules, parenteral solutions and suppositories incorporating same.

Example

The hydrochloride salt of the compound of formula (I) is administered orally as a tablet to a patient identified by a clinician as having the symptoms associated with inhibited sexual desire or other psychosexual dysfunction. The patient is administered a daily dose of 100 mg (calculated as base) in two or three equally divided doses, 6 hours between doses.

The patient is treated continuously for several months and then taken off the drug periodically to determine whether the underlying pathology is resolved. If not, treatment is reinstituted.

## Claims

1. Use of *m*-chloro-α-*t*-butylaminopropiophenone of formula (I),

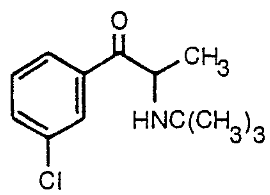

(I)

or a pharmaceutically acceptable salt thereof, for the manufacture of a medicament for the treatment of psychosexual dysfunction in a human being.

2. Use according to claim 1 of a pharmaceutically acceptable salt of *m*-chloro-α-*t*-butylaminopropiophenone.

3. Use according to claim 1 of *m*-chloro-α-*t*-butylaminopropiophenone hydrochloride.

4. Use of a compound defined in any of claims 1 to 3 for the manufacture of a medicament according to claim 1, suitable for oral administration.

5. Use of a compound defined in any of claims 1 to 3 for the manufacture of a medicament according to claim 1, suitable for parenteral administration.

6. Use of a compound defined in any of claims 1 to 3 for the manufacture of a medicament according to claim 1, suitable for rectal administration.

7. Use of a compound defined in any of claims 1 to 3 for the manufacture of a medicament according to claim 1 in unit dosage form.

8. Use of a compound defined in any of claims 1 to 3 for the manufacture of a medicament according to claim 1 in the form of a tablet suitable for oral administration.

9. Use of a compound defined in any of claims 1 to 3 for the manufacture of a medicament according to claim 1 in the form of a capsule suitable for oral administration.

10. Use of a compound defined in any of claims 1 to 3 for the manufacture of a medicament according to claim 1 in the form of a suppository suitable for rectal administration.

## Patentansprüche

1. Verwendung von m-Chlor-α-tert.-butylaminopropiophenon der Formel I

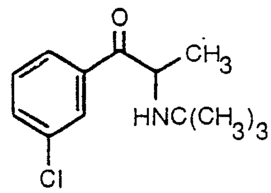

(I)

oder eines pharmazeutisch verträglichen Salzes davon, zur Herstellung eines Medikaments zur Behandlung von psychosexuellen Funktionsstörungen beim Menschen.

2. Verwendung nach Anspruch 1 eines pharmazeutisch verträglichen Salzes vom m-Chlor-α-tert.-butylaminopropiophenon.

3. Verwendung nach Anspruch 1 von m-Chlor-α-tert.-butyl-aminopropiophenon.Hydrochlorid.

4. Verwendung einer Verbindung gemäß einem der Ansprüche 1 bis 3 zur Herstellung eines Medikaments gemäß Anspruch 1, geeignet für orale Verabreichung.

5. Verwendung einer Verbindung gemäß einem der Ansprüche 1 bis 3 zur Herstellung eines Medikaments gemäß Anspruch 1, geeignet für parenterale Verabreichung.

6. Verwendung einer Verbindung gemäß einem der Ansprüche 1 bis 3 zur Herstellung eines Medikaments gemäß Anspruch 1, geeignet für rektale Verabreichung.

7. Verwendung einer Verbindung gemäß einem der Ansprüche 1 bis 3 zur Herstellung eines Medikaments gemäß Anspruch 1 in Einheitsdosis-Form.

8. Verwendung einer Verbindung gemäß einem der Ansprüche 1 bis 3 zur Herstellung eines Medikaments gemäß Anspruch 1 in Form einer Tablette, geeignet für orale Verabreichung.

9. Verwendung einer Verbindung gemäß einem der Ansprüche 1 bis 3 zur Herstellung eines Medikaments gemäß Anspruch 1 in Form einer Kapsel, geeignet für orale Verabreichung.

10. Verwendung einer Verbindung gemäß einem der Ansprüche 1 bis 3 zur Herstellung eines Medikaments gemäß Anspruch 1 in Form eines Suppositoriums, geeignet für rektale Verabreichung.

## Revendications

1. Utilisation de la *m*-chloro-α-*t*-butylaminopropiophénone de formule (I)

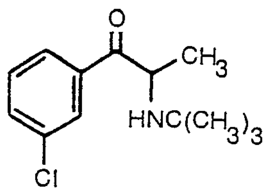

(I)

ou d'un sel pharmaceutiquement acceptable de celle-ci, pour la préparation d'un médicament pour le traitement d'une dysfonction psychosexuelle chez l'être humain.

2. Utilisation suivant la revendication 1 d'un sel pharmaceutiquement acceptable de la *m*-chloro-α-*t*-butylaminopropiophénone.

3. Utilisation suivant la revendication 1 du chlorhydrate de *m*-chloro-α-*t*-butylaminopropiophénone.

4. Utilisation d'un composé défini dans l'une quelconque des revendications 1 à 3 pour la préparation d'un médicament suivant la revendication 1 propre à l'administration par voie orale.

5. Utilisation d'un composé défini dans l'une quelconque des revendications 1 à 3 pour la préparation d'un médicament suivant la revendication 1 propre à l'administration par voie parentérale.

6. Utilisation d'un composé défini dans l'une quelconque des revendications 1 à 3 pour la préparation d'un médicament suivant la revendication 1 propre à l'administration par voie rectale.

7. Utilisation d'un composé défini dans l'une quelconque des revendications 1 à 3 pour la préparation d'un médicament suivant la revendication 1 sous forme dosée unitaire.

8. Utilisation d'un composé suivant l'une quelconque des revendications 1 à 3 pour la préparation d'un médicament suivant la revendication 1 sous la forme d'un comprimé propre à l'administration par voie orale.

9. Utilisation d'un composé suivant l'une quelconque des revendications 1 à 3 pour la préparation d'un médicament suivant la revendication 1 sous la forme d'une capsule propre à l'administration par voie orale.

10. Utilisation d'un composé suivant l'une quelconque des revendications 1 à 3 pour la préparation d'un médicament suivant la revendication 1 sous la forme d'un suppositoire propre à l'administration par voie rectale.